# EUROPEAN PATENT APPLICATION

(11) **EP 0 565 317 A1**
(43) Date of publication of application: **13.10.1993**
(21) Application number: 93302617.1
(22) Date of filing: 02.04.1993
(51) Int. Cl.: C07C 217/18, A61K 31/135, C07D 263/22

(54) **(2-(2-hydroxy-2 phenylethylamino)ethoxy) phenyl derivatives, processes and intermediates for their preparation, pharmaceutical compositions containing them and their use as Beta-3-adrenoceptor agonists**

(30) Priority: 10.04.1992 GB 9207964
(71) Applicant: ZENECA LIMITED, London W1Y 6LN (GB)
(72) Inventor: Holloway, Brian Roy, Macclesfield, Cheshire, SK10 4TG (GB); Howe, Ralph, Macclesfield, Cheshire, SK10 4TG (GB); Lecount, David James, Macclesfield, Cheshire, SK10 4TG (GB); Rao, Balbir Singh, Macclesfield, Cheshire, SK10 4TG (GB)
(74) Representative: Denerley, Paul Millington

(57) **Abstract**

CHEMICAL COMPOUNDS 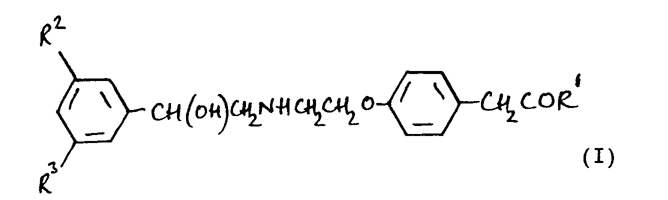
wherein R¹ is C₁₋₈alkyl optionally interrupted by an oxygen atom ; R² is hydrogen, fluoro, bromo, chloro, methyl or trifluoromethyl ; R³ is hydrogen or fluoro and in vivo hydrolysable esters and pharmaceutically acceptable salts thereof are described as (β₃-adr̅e̅no̅c̅e̅p̅tor agonists having anti-obesity and related therapeutic utilities. Processes for their preparation are described as are compositions containing them.

## Description

The present invention relates to 2-hydroxy-2-phenylethylamino compounds and in particular to such compounds containing a phenylacetyl group. This invention further relates to processes and intermediates for their preparation, to their use in methods of therapy and to pharmaceutical compositions containing them. The compounds of the present invention are (β₃-adrenoceptor agonists and are of value in disease conditions mediated through such adrenoceptors. Administration of the compounds of this invention to warm-blooded animals provides a thermogenic effect, that is thermogenesis is stimulated and administration of the compounds is of use, for example, in the treatment of obesity and related conditions such as mature onset diabetes associated with obesity. In addition, the compounds of this invention improve glucose tolerance in warm-blooded animals and are of use in combatting disease conditions wherein such activity is beneficial for example they have hypoglycaemic activity. The compounds of this invention may also be used in the treatment of non-insulin dependent diabetes mellitus (NIDDM) and in conditions wherein insulin resistance is of importance such as hypertension, hyperlipidaemia and decreased fibrinolysis (Reaven's syndrome or Syndrome X).

European Patent Application 21636, published 7 July 1981, discloses compounds of the formula: 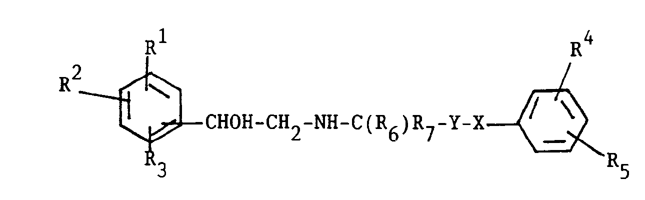
or a pharmaceutically acceptable salt thereof, in which R₁ is a hydrogen, fluorine, chlorine or bromine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group; R₂ is a hydrogen, fluorine, chlorine or bromine atom or hydroxyl group; R₃ is a hydrogen, chlorine or bromine atom ora hydroxyl group, R₄ is an alkyl group of 1 to 10 carbon atoms substituted by a hydroxyl, lower alkoxyl, oxo, lower acyloxy or OCH₂CO₂H group or lower alkyl ester thereof; R₅ is a hydrogen, chlorine or fluorine atom or a methyl, methoxyl or hydroxyl group or a carboxylic acid group or a salt, ester or amide thereof; R₆ is a hydrogen atom or a methyl, ethyl or propyl group; R₇ is a hydrogen atom or methyl, ethyl or propyl group; X is an oxygen atom or bond; and Y is an alkylene group of up to carbon atoms ora bond. These compounds are stated to possess anti-obesity and/or anti-hyperglycaemic activity.

We have now discovered a small class of compounds which provide significant thermogenic effects at doses which cause relatively few side-effects. It is understood that selectivity of thermogenic effect, for example lack of cardiac side-effects, is an important requirement for a useful therapeutic agent in the treatment of, for example, obesity and related conditions.

Accordingly the present invention provides a compound of the formula (I): 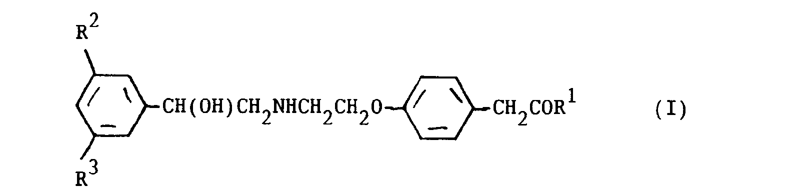
wherein R¹ is C₁₋₈ alkyl optionally interrupted by an oxygen atom;
R² is hydrogen, fluoro, bromo, chloro, methyl or trifluoromethyl;
R³ is hydrogen or fluoro;
or an in vivo hydrolysable ester or a pharmaceutically acceptable salt thereof.

In one aspect R¹ is C₁₋₈ alkyl for example methyl, ethyl, isopropyl, n-propyl, n-butyl, n-hexyl or n-octyl.

In another aspect R¹ is C₁₋₆ alkyl substituted by methoxy orethoxy, for example R¹ is methoxymethyl, methoxyethyl, methoxypropyl, methoxybutyl, ethoxymethyl, ethoxyethyl and ethoxypropyl.

In a further aspect R¹ is C₁₋₅alkyl, preferably methyl, substituted by propoxy preferably n-propoxy.

In yet a further aspect R¹ is C₁₋₄ alkyl substituted by a C₄₋₇ alkoxy group (the total number of carbon atoms being not more than eight) for example R¹ is butoxymethyl, pentoxymethyl, butoxyethyl, hexoxyethyl and hep- toxymet hyl.

Preferably the alkyl groups are straight-chained.

Preferred groups R¹ include methyl, ethyl, n-propyl, methoxymethyl, n-propoxymethyl and methoxypropyl. A particular preferred group R¹ is methoxymethyl.

Suitable groups R² are hydrogen, fluoro, chloro, bromo and trifluoromethyl. Of these hydrogen, chloro and trifluoromethyl are preferred.

The compounds of the formula (I) are basic and may be isolated and used either in the form of the free base or of a pharmaceutically acceptable acid-addition salt thereof. Particular examples of pharmaceutically acceptable acid-addition salts include, for example, salts with inorganic acids such as hydrohalides (especially hydrochlorides or hydrobromides), sulphates and phosphates, and salts with organic acids such as succinates, citrates, lactates,tartrates, oxalates and salts derived from acidic water-soluble polymers.

In vivo hydrolysable esters may be formed at the hydroxy group (-OH). In vivo hydrolysable esters are those pharmaceutically acceptable esters that hydrolyse in the human or animal body to produce the parent hydroxy compound. Such esters can be identified by administering, for example intravenously to a test animal, the compound under test and subsequently examining the test animal's body fluids. Suitable in vivo hydrolysable esters for hydroxy include as their acyl group for example acetyl, propionyl, pivaloyl, C1-4alkoxycarbonyl for example ethoxycarbonyl and phenylacetyl.

It will be appreciated that the compounds of the formula (I) contain one or more asymmetric carbon atoms and can exist as optically active enantiomers or as optically inactive racemates. The present invention encompasses any enantiomer, racemate and/or (when two or more asymmetric carbon atoms are present) diastereoisomer, which when administered in a therapeutic amount provides a thermogenic effect in warm-blooded animals, it being well known in the chemical art how to prepare individual enantiomers, for example by resolution of the racemate or by stereospecific synthesis, and how to determine the thermogenic properties, for example, using the standard tests described hereinafter. It is preferred that the compounds of the present invention are provided in the (R) absolute configuration at the -CH(OH)- group (under the Cahn-Prelog-Ingold rules) which generally corresponds to the laevorotatory optically active form (-) in this class of compounds.

Particular compounds of the present invention are:
1-[4-(2-(2-hydroxy-2-phenyiethyiamino)ethoxy)phenyi]-3-methoxypropan-2-one;
1-[4-(2-(2-hydroxy-2-phenylethylamino)ethoxy)phenyl]-3-n-propoxypropan-2-one;
1-[4-(2-(2-hydroxy-2-(3-chlorophenyl)ethylamino)ethoxy)phenyl]-3-methoxypropan-2-one;
1-[4-(2-(2-hydroxy-3-(3-chlorophenyl)ethylamino)ethoxy)phenyl]-3-n-propoxypropan-2-one; and
1-[4-(2-(2-hydroxy-2-(3-trifluoromethylphenyl)ethylamino)ethoxy)phenyl-3-methoxypropan-2-one.

In order to use a compound of the present invention or an in vivo hydrolysable ester or pharmaceutically acceptable salt thereof for the therapeutic treatment of warm-blooded mammals including humans, in particular for treating obesity, it is normally formulated in accordance with standard pharmaceutical practice as a pharmaceutical composition.

Therefore in another aspect the present invention provides a pharmaceutical composition which comprises a compound of the formula (I) or an in vivo hydrolysable ester or a pharmaceutically acceptable salt thereof and a pharmaceutically acceptable carrier.

The pharmaceutical compositions of this invention may be administered in standard manner for example by oral or parenteral administration. For these purposes they may be formulated by means known to the art into the form of, for example, tablets, capsules, pills, powders, aqueous or oily solutions or suspensions, emulsions, and sterile injectable aqueous or oily solutions or suspensions.

In general compositions for oral administration are preferred.

The compositions may be obtained using standard excipients and procedures well known in the art. A unit dose form such as a tablet or capsule will usually contain, for example 0.1-500 mg of active ingredient, more suitably 10-250 mg, and preferably 50-100 mg of the compound of this invention. The compositions may also contain other active ingredients known for use in the disease condition to be treated, for example appetite suppressants, vitamins, antihypertensives and hypoglycaemic agents such as sulphonylureas, biguanides and thiazolidinediones. It is understood that such compositions cover co-formulation, concurrent and sequential therapy of the two or more active ingredients.

In one aspect of the present invention, a compound of the formula (I) may be formulated for oral administration in a sustained (or delayed) release composition, for example a matrix tablet formulation comprising insoluble or swellable polymeric filler, or a coated spheroid formulation.

When used to produce thermogenic effects in warm-blooded animals including man, a compound of the formula (I) or an in vivo hydrolysable ester or pharmaceutically acceptable salt thereof as appropriate, will be administered so that a dose in the general range 0.002-20 mg/kg, suitably in the range 0.02-10 mg/kg, and preferably in the range 0.5-5 mg/kg is administered daily, given in a single dose or divided doses as necessary. However, it will be appreciated by those skilled in the art that dosage will necessarily be varied as appropriate, depending on the severity of the condition under treatment and on the age and sex of the patient and according to known medical principles.

In addition the compounds of the present invention lower triglyceride levels and cholesterol levels and raise high density lipoprotein levels and are therefore of use in combating medical conditions wherein such lowering (and raising) is thought to be beneficial. Thus they may be used in the treatment of hypertriglyceridaemia, hy- percholesterolaemia and conditions of low HDL (high density lipoprotein) levels in addition to the treatment of atherosclerotic disease such as of coronary, cerebrovascular and peripheral arteries, cardiovascular disease and related conditions.

Accordingly in another aspect the present invention provides a method of lowering triglyceride and/or cholesterol levels and/or increasing high density lipoprotein levels which comprises administering, to an animal in need thereof, a therapeutically effective amount of a compound of the formula (I) or in vivo hydrolysable ester or pharmaceutically acceptable salt thereof. In a further aspect the present invention provides a method of treating atherosclerosis which comprises administering, to an animal in need thereof, a therapeutically effective amount of a compound of the formula (I) or an in vivo hydrolysable ester or pharmaceutically acceptable salt thereof. The compositions are formulated and administered in the same general manner as detailed above for producing a thermogenic effect. They may also contain other active ingredients known for use in the treatment of atherosclerosis and related conditions, for example fibrates such as clofibrate, bezafibrate and gem- fibrozil; inhibitors of cholesterol biosynthesis such as HMG-CoA reductase inhibitors for example lovastatin, simvastatin and pravastatin; inhibitors of cholesterol absorption for example beta-sitosterol and (acyl CoA:cholesterol acyltransferase) inhibitors for example melinamide; anion exchange resins for example cholestyramine, colestipol or a dialkylaminoalkyl derivatives of a cross-linked dextran; nicotinyl alcohol, nicotinic acid or a salt thereof; vitamin E; and thyromimetics.

In a further aspect the compounds of the present invention stimulate the "atypical" p-adrenoceptors in the gastrointestinal tract and therefore inhibit gastrointestinal motility. They may be of use in combatting medical conditions wherein stimulation of "atypical" (3-adrenoceptors in the gastrointestinal tract is thought to be beneficial, such as in combatting medical conditions wherein inhibition of gastrointestinal motility is thought to be of value. Thus they may be of use for example in the treatment of inflammatory bowel disease (IBD) (such as Crohn's disease and ulcerative colitis), irritable bowel syndrome (IBS), non-specific diarrhoea and dumping syndrome.

Accordingly the present invention provides a method of stimulating the "atypical" (3-adrenoceptors in the gastrointestinal tract which comprises administering, to an animal in need thereof, a therapeutically effective amount of a compound of the present invention.

In a further aspect the present invention provides methods of inhibiting gastrointestinal motility, treating IBD, treating IBS, treating non-specific diarrhoea and treating gastric emptying in umping syndrome which comprise administering to an animal in need thereof, a therapeutically effective amount of a compound of the present invention.

In a further aspect the present invention provides a process for preparing a compound of the formula (I) or an in vivo hydrolysable ester or a pharmaceutically acceptable salt thereof which process comprises:
a) reacting a compound of the formula (II) or (III) with a compound of the formula (IV): 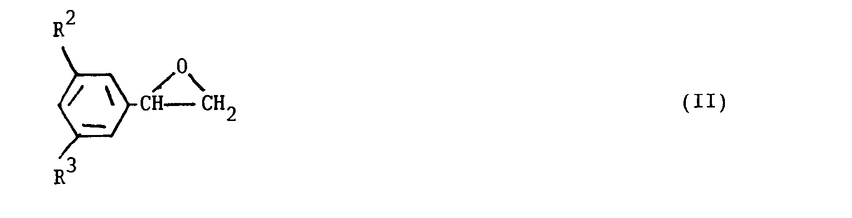 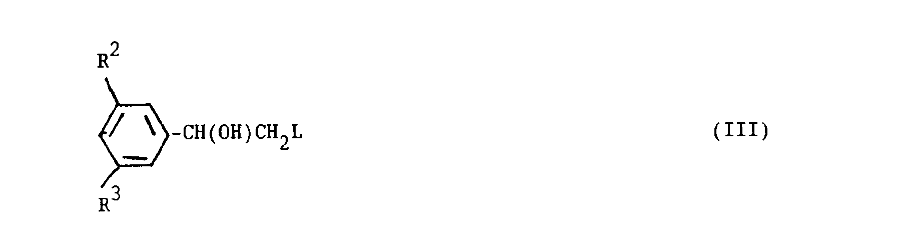 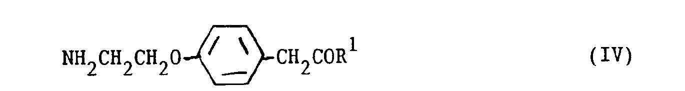
   wherein R¹, R² and R³ are as hereinbefore defined and L is a displaceable group; or
b) hydrolysis of a compound of the formula (V): 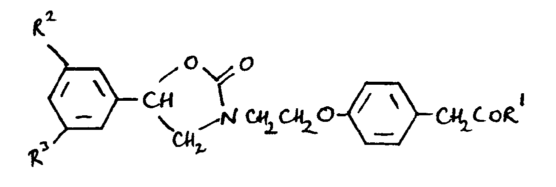
   wherein R¹, R² and R³ are as hereinbefore defined; or
c) reacting a compound of the formula (VI) with a compound of the formula (VII): 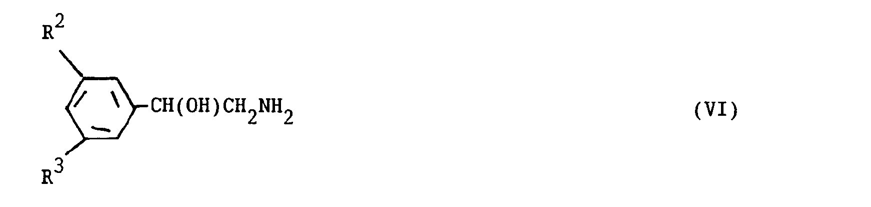 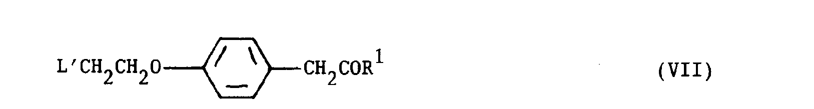
   wherein R¹, R² and R³ are as hereinbefore defined and L' is a leaving group; or
d) deprotecting a compound of the formula (VIII): 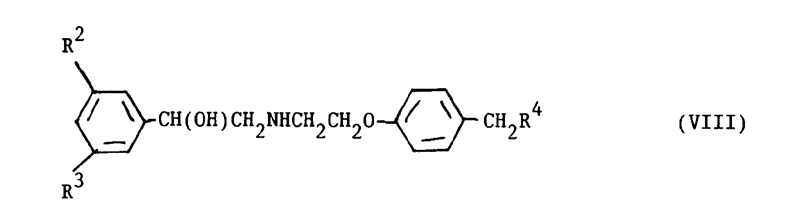
   wherein R⁴ is a protected derivative of a group -COR¹ and R² and R³ are as hereinbefore defined;
   and wherein any functional group is optionally protected and thereafter if necessary;
      (i) remoning any protecting groups;
      (ii) forming a pharmaceutically acceptable salt.

Protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting grou p in question, such methods being chosen so as to effect removal of the protecting group with minimum disturbance of groups elsewhere in the molecule.

A particular protecting group is a hydrogenolysable group present on the nitrogen atom of -CH(OH) CH₂NCH₂CH₂0-. Suitably the protecting group is benzyl ora substituted benzyl group. Such a protecting group may be removed in conventional manner using methods of catalytic hydrogenation, for example palladium on carbon catalysts in an atmosphere of hydrogen. Suitable conditions include ambient and elevated temperatures and pressures in a solvent such as a C₂₋₆-alkanol for example ethanol or propan-2-ol. Compounds corresponding to formula (I) protected with a hydrogenolysable group on the nitrogen atom may be prepared by methods analogous to those described above for formula (I).

The reaction between a compound of the formulae (II) or (III) and a compound of the formula (IV) may be performed in a suitable solvent for example an alcohol such as ethanol or propan-2-ol, at a temperature in the range for example 10°C to 110°C and most conveniently at or near the boiling-point of the reaction mixture. In the compound of the formula (III) L may be for example halogen such as chloro or bromo or an aryl sulphonyloxy such as toluenesulphonyloxy or an alkane sulphonyloxy group such as methanesulphonyloxy.

The compounds of the formula (IV) are prepared in any convenient manner known to those skilled in the art. For example they may be conveniently prepared by reacting compound (IX) with a compound of the formula (X):

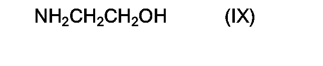

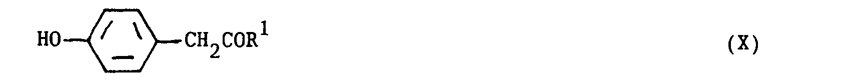

For example this reaction may be performed using the Mitsunobu reaction with diethyl azodicarboxylate and triphenylphosphine. Desirably the amino function is protected during this reaction and subsequently deprotected in conventional manner. Examples of suitable protecting groups for the amino function include the phthaloyl and t-butoxycarbonyl groups. The compounds of the formula (X) may be prepared according to the methods described in the Example hereinafter.

The compound of the formula (V) may be hydrolysed to a compound of the formula (I) under conditions known in the beta-blocker art; for example via alkaline hydrolysis in a suitable solvent.

The compounds of the formula (V) may be prepared by the reaction of a compound of the formula (X) with a compound of the formula (XI): 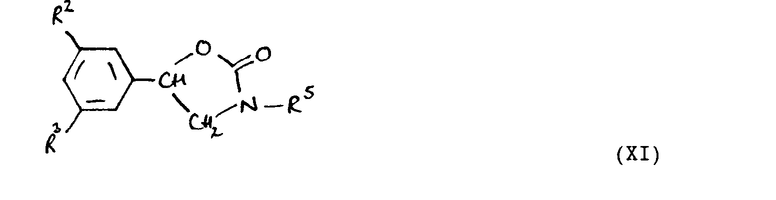
wherein R⁵ is a group -CH₂CH₂0H. This reaction may be performed in any conventional manner for example by a method analogous to the reaction of the compounds of the formulae (IX) and (X). In an alternative the compounds of the formula (V) may be prepared by the reaction of a compound of the formula (XI) wherein R⁵ is hydrogen with a compound of the formula (VII) as hereinbefore described. In a further alternative the compounds of the formula (V) may be prepared by the reaction of a compound of the formula (II) with a compound of the formula (XII): 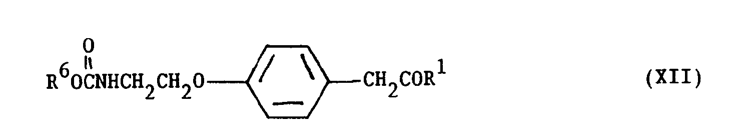
wherein R¹ is as hereinbefore defined and R⁶0- is a leaning group, for example R⁶0- is C₁₋₄alkoxy.

The compound of the formula (XI) wherein R⁵ is -CH₂CH₂0H may be prepared for example by reaction of a compound of the formula (II) with an N-alkoxycarbonyl derivative of a compound of the formula (IX) wherein the hydroxy group is optionally protected, for example the tetrahydropyranyl ether of t-butoxycarbonylaminoethanol. The compounds of the formula (XI) wherein R⁵ is hydrogen are obtainable in conventional manner. The compounds of the formulae (VII) and (XII) may be obtained by alkylation of the compounds of the formula (X) in conventional manner.

The reaction between the compounds of the formulae (VI) and (VII) is conveniently performed under conditions analogous to the reaction between compounds of the formulae (III) and (IV). L'may have similar values as recited hereinabove for L.

In the deprotection of a compound of the formula (VIII) R⁴ may for example be an acetal such as 2-methyl-1,3-dioxolan-2-yl, 2-methoxymethyl-1,3-dioxolan-2-yl or 2-methoxypropyl-1,3-dioxolan-2-yl. Such groups may be readily converted to a group -COR¹ by acid hydrolysis. The compounds of the formula (VIII) may be prepared by methods analogous to those described herein for the preparation of the compounds of the formula (I).

In vivo hydrolysable esters may be prepared in conventional manner for example by reacting the hydroxy group with an activated derivative of an acid.

Pharmaceutically acceptable salts may be prepared by reacting the compound of the formula (I) with the appropriate acid in conventional manner. Alternatively when a hydrogen halide salt is required, it may conveniently be obtained by hydrogenation of the free base togetherwith a stoichiometric amount of the corresponding benzyl halide.

The following biological test methods, data and Examples serve to illustrate this invention.

The thermogenic effects of the compound of the formula (I) may be demonstrated using one or more of the following standard tests:-
(a) Rats are cold adapted at 4°C for 5 days to increase their capacity for thermogenesis. They are then transferred to a warm environment of 25°C for 2 days. On the following day, a test compound is administered sub-cutaneously ororally. Animals are sacrificed one hour later and the interscapular, brown adipose tissue (BAT) pad is removed. BAT mitochondria are prepared by differential centrifugation and GDP binding is determined (Holloway et al., International Journal of Obesity, 1984, 8, 295) as a measure of thermogenic activation. Each test includes a control which is dosed with the solution/suspension vehicle only and a positive control which is dosed with isoprenaline (as its sulphate) at 1mgkg-¹. Test compounds are routinely dosed at 0.1, 0.3, 1.0, 3.0, and 10 mgkg-¹ and results expressed in terms of the effect on GDP binding produced by the positive control. From these results, a dose (ED₅₀) necessary to produce 50% of the isoprenaline effect is calculated by curve fitting. Compounds are considered active in this test if they cause a significant elevation in GDP binding as compared to controls.
(b) Rats are adapted to a thermoneutral environment (29°C) for 2 weeks in order to decrease their capacity for BAT mediated non-shivering thermogenesis. During the final 5 days the animals are trained to use an apparatus for measuring heart rate non-invasively via foot-pad electrodes connected to an ECG integrator giving a continuous read-out of heart rate. A test compound is administered sub-cutaneously or orally at the ED₅₀ determined in test (a), and heart rate is determined 15-30 minutes after dosing. The procedure is then repeated in subsequent tests using increasing multiples of the ED₅₀ determined in test (a) until the heart rate (HR) reaches or exceeds 500 beats per minute, or until the dose reaches 100 times the ED₅₀ determined in test (a). The dose necessary to produce a heart rate of 500 beats per minute (D₅₀₀ dose) is estimated.
   The ratio of D₅₀₀ to ED₅₀ in test (a) can be defined as the selectivity index (SI) and provides a measure of the selectivity of the compound for BAT as opposed to the cardiovascular system. Compounds are considered to have significant selectivity which have an SI of >1. Non-selective compounds have an SI of <1 (for example isoprenaline = 0.06).
(c) Rats are kept at 23°C for at least two days, then fasted overnight. On the following day, the basal metabolic rate of the animals is determined using a close-circuit oxygen consumption apparatus of the type described by Arundel et al., 1984, J. Appl. Physiol. Respirat. Environ. Exercise Physiol., 1984, 57 (5) 1591-1593. The rats are then dosed (orally) with test compound at about 1 mgkg-¹ as a solution or suspension in 0.025% w/v Polysorbate 80 (0.5ml/100g). Metabolic rate is then determined for at least one hour after dosing. Compounds are considered active in this test if they cause a significant increase in metabolic rate as compared to control animals (Student's t test: p <0.05) dosed only the solution or suspension vehicle.

In the above tests, the compounds of the formula (I) in general produce effects of the following order without producing overt toxicity:-
test (a): sub-cutaneous or oral ED₅₀ for GDP binding in BAT mitochondria of 0.01-1 0 mgkg⁻¹;
test (b): show an SI of >50; and
test (c): show 2-9ml 0₂ min⁻¹(Kg^{0.75})⁻¹ at 1mgkg⁻¹ p.o.

By way of illustration, the compound described in the accompanying Example 1, produces the following effects in the above tests:-
(a) oral ED₅₀ 0.98mgkg-¹;
(b) SI >50 (oral); D₅₀₀:>49.3 mgkg-¹ (oral)
(c) 4.99 ml O₂ min⁻¹ (Kg^{0.75})⁻¹ at 1mgkg⁻¹ p.o.

The invention will now be illustrated by the following Example in which, unless otherwise stated:
a) nuclear magnetic response (NMR) spectra were determined at 200MHz in dₛ-dimethylsulphoxide/d₄- acetic acid as solvent unless otherwise stated, using tetramethylsilane (TMS) as an internal standard and are expressed in delta values (parts per million) for protons relative to TMS, using conventional abbreviations to describe signal types.
b) chromatography was performed on Merck Kieselgel (Art 9385; 230-400 Mesh) obtainable from E. Merck, Darmstadt, Federal Republic of Germany.
c) where noted, solutions were taken to pH values as judged by moist indicator paper.
d) evaporations were carried out under reduced pressure using a rotary evaporator.
e) melting-points are uncorrected.

### EXAMPLE 1

### 1-[4-(2-(2-Hydroxy-2-phenylethylamino)ethoxy)phenyl]-3-methoxypropan-2-one

A solution of 3-[2-(4-(3-methoxy-2-oxopropyl)phenoxy)ethyl]-5-phenyloxazolidin-2-one (900mg) in hot ethanol (22ml) was treated with 2N aqueous sodium hydroxide (3.7ml) and the reaction mixture was stirred under reflux for 1 hour under argon.The reaction mixture was cooled, poured into water(100ml)and the product was extracted into dichloromethane (3 x 100ml). The dichloromethane extracts were washed with brine (50ml), dried, and the solvent was removed under reduced pressure. The crude product was purified by column chromatography using methanol in dichloromethane as eluant (gradient elution; 2.5-3.5% methanol). The appropriate fractions were combined and the solvent removed under reduced pressure. The residual solid (350mg) was dissolved in ethanol (6ml) and treated with concentrated hydrochloric acid (0.09ml) to yield white crystals of the title compound as the hydrochloride (140mg); m.p. 141-143°C; microanalysis: found C, 63.3; H, 6.8; N, 3.8%; required for C₂ₒH₂₆CLN0₄: C, 63.2; H, 6.9; N, 3.7%; NMR 3.09-3.52 (m, 4H, CH₂NHCH₂), 3.31 (s, 3H, CH₃), 3.69 (s, 2H ArCH₂), 4.12 (s, 2H, COCH₂O), 4.27-4.37 (m, 2H, CH₂OAr), 5.00-5.09 (m, 1 H, CHOH), 6.92-7.42 (m, 9H, aromatic H).

The starting material was prepared as follows:
a) A solution of N-t-butoxycarbonylaminoethanol (22g), dihydropyran (19ml) and pyridinium p-toluenesulphonate (3.4g) in anhydrous dichloromethane (900ml) was left for 16 hours at 22°C. Ether (1500ml) was added, the solution was washed with water (2 x 500ml), dried, and the solvent removed under reduced pressure to give 2-(2-(N-t-butoxycarbonylamino)ethoxy)tetrahydropyran (33g) as a brown oil.
b) The product from a) above (25.5g) was added to a stirred suspension of sodium hydride (4.17g of 60% dispersion in mineral oil; prewashed with hexane) and styrene oxide (11.9ml) in anhydrous dimethylformamide (400ml) under argon. The reaction mixture was stirred at 37-40°C for 2 hours, then cooled in an ice bath and water (40ml) was added. The mixture was poured into water (2000ml) and the product extracted into ethyl acetate (700ml, 500ml, 300ml). The combined ethyl acetate extracts were washed with brine (600ml), dried and the solvent removed under reduced pressure. The residue was subjected to chromatography using ethyl acetate in hexane as eluant (gradient elution 50-55% ethyl acetate). The appropriate fractions were combined to yield 5-phenyl-3-(2-(tetrahydropyran-2-yloxy)ethyl)oxazolidin-2-one (7.8g) as a colourless oil.
c) A solution of the product from b) above (7.8g) and pyridinium p-toluenenesulphonate (0.6g) in ethanol (200ml) was stirred at 70°C for 2 hours. The ethanol was removed under reduced pressure and the residue partitioned between water (250ml) and ethyl acetate (250ml). The organic layer was separated and the aqueous layer re-extracted with ethyl acetate. The ethyl acetate extracts were washed with water (100mi), dried, and the solvent removed under reduced pressure. The residue was subjected to chromatography using ethyl acetate as eluant. The appropriate fractions were combined to yield 5-phenyl-3-(2-hydroxyethyl)oxazolidin-2-one (4.5g) m.p. 47-49°C (ethyl acetate/cyclohexane); microanalysis: found C, 63.6; H, 6.3: N, 6.7%; required for C₁₁H₁₃NO₃: C, 63.8; H, 6.3; N, 6.8%.
d) Diethylazodicarboxylate (1.14ml) was added dropwise to a stirred solution of 1-(4-hydroxyphenyl)-3-methoxypropan-2-one (1.3g), 5-phenyl-3-(2-hydroxyethyl)oxazolidin-2-one (1.5g), and triphenylphosphine (1.9g) in anhydrous tetrahydrofuran (38ml) at ice-bath temperature underargon. The reaction mixture was left for 64 hours at 20°C and then the solvent was removed under reduced pressure. The residue was dissolved in dichloromethane and the solution seeded with 1,2-dicarbethoxyhydrazine. The solution was allowed to stand for 1 hour at ice-bath temperature, the solid was collected and the filtrate was subjected to column chromatography using ethyl acetate in hexane as eluant (gradient elution; 45-60% ethyl acetate). The appropriate fractions were combined to yield 3-[2-(4-(3-methoxy-2-oxopropyl)phenoxy)ethyl]-5-phe- nyloxazolidin-2-one (1.10g) as an oil.

### 1-(4-Hydroxyphenyl)-3-methoxypropan-2-one was prepared as follows:

A solution of methoxyacetonitrile (3.1 ml) in anhydrous tetrahydrofuran (10 ml) was added dropwise to a stirred solution of 4-benzyloxybenzyl magnesium chloride (prepared from benzyloxybenzyl chloride (11.6 g) and magnesium (1.2 g)) in tetrahydrofuran (100 ml) cooled to -10°C under argon. The reaction mixture was stirred for 2 hours at 20°C. Ice cold water (70 ml), followed by 12 N sulphuric acid (14 ml), was added, and the mixture was stirred for 20 minutes. The product was extracted into ether (2 x 50 ml). The ether extracts were washed with aqueous sodium carbonate (50 ml), washed with water (50 ml), dried and the solvent removed under reduced pressure. The residue was purified by column chromatography using 25% ethyl acetate in hexane as eluant. The appropriate fractions were combined and evaporated to yield 1-(4-benzyloxyphenyl)-3-me- thoxypropan-2-one (3.2 g) m.p. 47-49°C (cyclohexane); microanalysis: found C, 75.2; H, 6.6% required for C₁₇H₁₈O_{3:} C, 75.6; H, 6.7%.

A stirred suspension of 5% palladium on carbon (1.4 g) in a solution of 1-(4-benzyloxyphenyl)-3-methox- ypropan-2-one (2.8 g) in cyclohexene (90 ml) and tetrahydrofuran (90 ml) was heated under reflux for 48 hours underargon. The reaction mixture was cooled and filtered. The filtrate was evaporated under reduced pressure and the residue purified by chromatography using 35% ethyl acetate in hexane as eluant. The appropriate fractions were combined and evaporated to yield 1-(4-hydroxyphenyl)-3-methoxypropan-2-one (1.3 g) as a colourless oil.

### EXAMPLES 2-5

Prepared in a manner similar to Example 1 were:
1-[4-(2-(2-hydroxy-2-phenylethylamino)ethoxy)phenyl]-3-n-propoxypropan-2-one hydrochloride, m.p. 155-157°C; microanalysis: found: C, 65.2; H, 7.5; N, 3.4%; C₂₂H₃₀CIN0₄ requires: C, 64.2; H, 7.4; N, 3.4%. NMR: 0.89 (t, 3H, CH₃-CH₂), 1.54 (sextet, 2H, CH₂-CH₂-CH₃), 3.38 (t, 2H, O-CH₂-CH₂), 3.06-3.48 (m, 4H, CH₂-NH-CH₂), 3.7 (s, 2H, Ar-CH₂), 4.15 (s, 2H, CO-CH₂-O), 4.31 (t, 2H, CH₂-O), 5.02 (dd, H, CH-OH), 6.92-7.42 (m, 9H, aromatic H);
1-[4-(2-(2-hydroxy-2-(3-chlorophenyl)ethylamino)ethoxy)phenyl]-3-methoxypropan-2-one hydrochloride, m.p. 119-120°C; microanalysis:
found: C, 58.1; H, 6.1; N, 3.3%; C₂₀H₂₄CINO₄ requires: C, 58.0; H, 6.1; N, 3.4%. NMR: 3.03-3.49 (m, 4H, CH₂-NH-CH2), 3.3 (s, 3H, CH₃), 3.69 (s, 2H, Ar-CH₂), 4.13 (s, 2H, CO-CH₂-O), 4.3 (m, 2H, CH₂-OAr), 5.01, (dd, 1 H, CH-OH), 6.9-7.56 (m, 8H, aromatic H);
1-[4-(2-(2-hydroxy-3-(3-chlorophenyl)ethylamino)ethoxy)phenyl]-3-n-propoxypropan-2-one hydrochloride, m.p.149-151°C; microanalysis:
found: C, 59.9; H, 6.8, N, 2.9%; C₂₂H₂₉CIN0₄ requires: C, 59.7; H, 6.6, N, 3.2%. NMR: 0.89 (t, 3H, CH₃), 1.54 (sextet, 2H, CH₂-CH₂-CH₃), 3.39 (t, 2H, O-CH₂-CH₂), 3.10-3.48 (m, 4H, CH₂-NH-CH₂), 3.7 (s, 2H, Ar-CH₂), 4.15 (s, 2H, CO-CH₂-O), 4.3 (t, 2H, CH₂-O), 5.05 (dd, 1 H, CH-OH), 6.9-7.5 (m, 8H, aromatic H); 1-[4-(2-(2-hydroxy-2-(3-trifluoromethylphenyl)ethylamino)ethoxy)phenyl-3-methoxypropan-2-one hydrochloride, m.p. 153-155°C;
microanalysis: found: C, 56.3; H, 5.7; N, 3.0%; C₂₁H₂₅CIF₃NO₄ requires: C, 56.3, H, 5.6; N, 3.1%; NMR: 3.3, (s, 3H, CH₃), 3.69, (s 2H, Ar-CH₂), 3.10-3.47 (m, 4H, CH₂-NH-CH₂), 4.13, (s, 3H, CO-CH₂-O), 4.25-4.35 (m, 2H, CH2-O-Ar), 5.13 (dd, 1 H, CH-OH), 6.9-7.8 (m, 8H, aromatic H).

Intermediates useful in the preparation of the starting materials for Examples 2-5 were obtained in a manner similar to that of Example 1:
5-(3-Chlorophenyl)-3-(tetrahydropyran-2-yloxy)ethyl)-oxazolidin-2-one and
5-(3-trifluoromethylphenyl)-3-(2-(tetrahydropyran-2-yloxy)ethyl)oxazolidin-2-one were obtained in a manner similar to Example 1 (b) as colourless oils.
5-(3-Chlorophenyl)-3-(2-hydroxyethyl)oxazolidin-2-one and
5-(3-trifluoromethyl)-3-(2-hydroxyethyl)oxazolidin-2-one were obtained in a manner similar to Example 1(c) but not further characterised.
3-[2-(4-(3-n-Propoxy-2-oxopropyl)phenoxy)ethyl]-5-phenyloxazolidin-2-one, m.p. 55-57°C (ethyl acetate/cyclohexane); microanalysis: found: C 69.4; H, 6.9; N, 3.5%; C₂₃H₂₇NO₅ requires: C, 69.4; H, 6.85; N, 3.5%;
3-[2-(4-(3-methoxy-2-oxopropyl)phenoxy)ethyl]-5-(3-chlorophenyl)oxazolidin-2-one as an oil;
3-[2-(4-(3-n-propoxy-2-oxopropyl)phenoxy)ethyl]-5-(3-chlorophenyl)oxazolidin-2-one, m.p. 66-68°C (ethyl acetate/cyclohexane); microanalysis: found: C, 64.4, H, 6.0; N, 3.2%; C₂₃H₂₆CIN0₅ requires: C, 64.0; H, 6.1; N, 3.2%; and
3-[2-(4-(3-methoxy-2-oxopropyl)phenoxy)ethyl]-5-(3-trifluoromethylphenyl)oxazolidin-2-one as an oil, were obtained in a manner similar to Example 1(d).
1-(4-Hydroxyphenyl)-3-n-propoxypropan-2-one, m.p. 47-49°C (cyclohexane); microanalysis: found C, 68.8; H, 7.8%; C₁₂H₁₆0₃ requires: C, 69.2; H,7.7% was prepared in a manner similar to that described above for 1-(4-hydroxyphenyl)-3-methoxypropan-2-one.

## Claims

1. A compound of the formula (I): 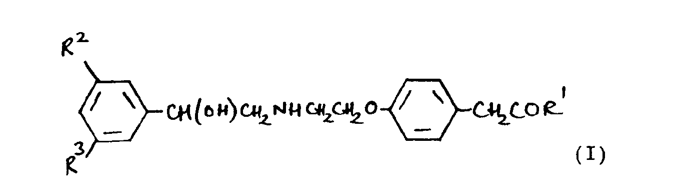
wherein R¹ is Cₗ₋₈alkyl optionally interrupted by an oxygen atom; R² is hydrogen, fluoro, bromo, chloro, methyl or trifluoromethyl; R³ is hydrogen or fluoro;
or an in vivo hydrolysable ester or a pharmaceutically acceptable salt thereof.

2. A compound according to claim 1 in the form of a salt formed with hydrochloric acid.

3. A compound according to either claim 1 or 2 wherein R¹ is methoxymethyl or n-propoxymethyl.

4. A compound according to any one of claims 1 to 3 wherein R² is hydrogen, chloro or trifluoromethyl and R³ is hydrogen.

5. A compound according to claim 1 which is:
1-[4-(2-(2-hydroxy-2-phenylethylamino)ethoxy)phenyl]-3-methoxypropan-2-one;
1-[4-(2-(2-hydroxy-2-phenylethylamino)ethoxy)phenyl]-3-n-propoxypropan-2-one;
1-[4-(2-(2-hydroxy-2-(3-chlorophenyl)ethylamino)ethoxy)phenyl]-3-methoxypropan-2-one;
1-[4-(2-(2-hydroxy-3-(3-chlorophenyl)ethylamino)ethoxy)phenyl]-3-n-propoxypropan-2-one; or
1-[4-(2-(2-hydroxy-2-(3-trifluoromethylphenyl)ethylamino)ethoxy)phenyl-3-methoxypropan-2-one.

6. A process for preparing a compound according to claim 1 which process comprises:
a) reacting a compound of the formula (II) or (III) with a compound of the formula (IV): 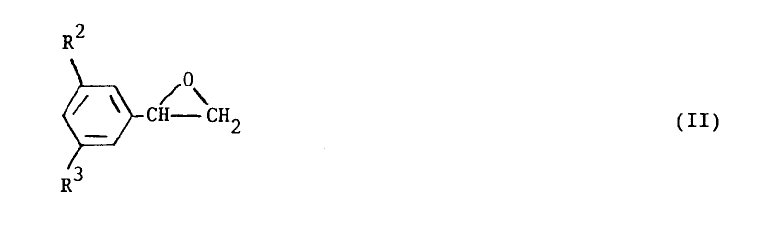 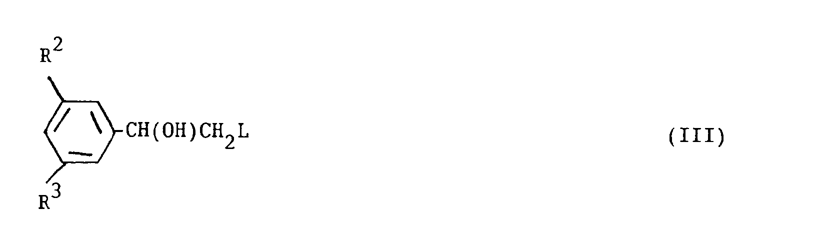 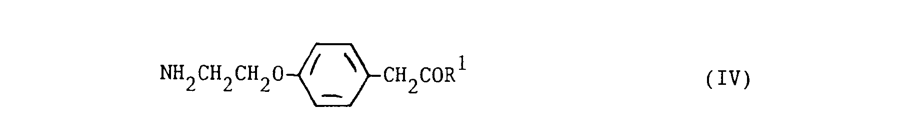
wherein R¹, R² and R³ are as defined in claim 1 and L is a displaceable group; or
b) hydrolysis of a compound of the formula (V): 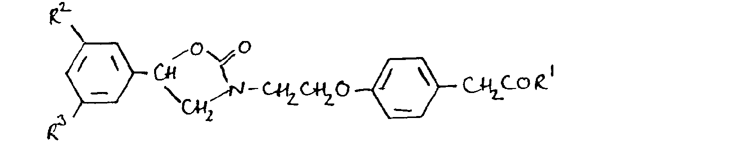
wherein R¹, R² and R³ are as hereinbefore defined; or
c) reacting a compound of the formula (VI) with a compound of the formula (VII): 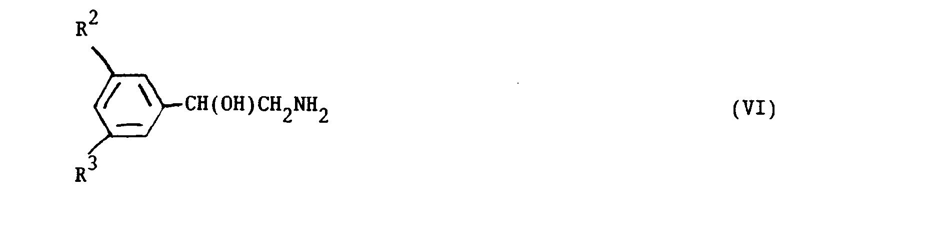 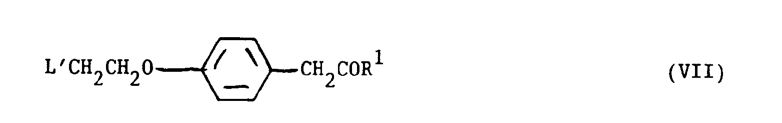
wherein R¹, R² and R³ are as hereinbefore defined and L' is a leaving group; or
d) deprotecting a compound of the formula (VIII): 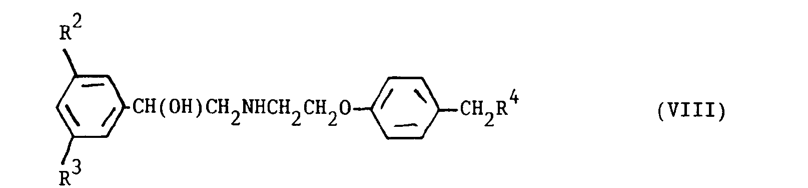
wherein R⁴ is a protected derivative of a group -COR¹ and R² and R³ are as hereinbefore defined;
and wherein any functional group is optionally protected and thereafter if necessary:
(i) removing any protecting groups
(ii) forming a pharmaceutically acceptable salt or in vivo hydrolysable ester.

7. A compound of the formula (IV), (V), (VII) or (VIII) as defined in claim 7.

8. The use of a compound according to any one of claims 1 to 5 for preparing a medicament for the treatment of disease conditions mediated through (β₃-adrenoceptors.

9. The use of a compound according to any one of claims 1 to 5 for preparing a medicament for the treatment of obesity or mature onset diabetes.
